(19) Europäisches Patentamt
European Patent Office
Office européen des brevets

(11) Publication number: **0 189 771 A2**

# (12) EUROPEAN PATENT APPLICATION

(21) Application number: 86100345.7

(22) Date of filing: 13.01.86

(51) Int. Cl.[4]: C07D 261/08 , A61K 31/42

(30) Priority: 22.01.85 US 693508

(43) Date of publication of application: 06.08.86 Bulletin 86/32

(84) Designated Contracting States: AT BE CH DE FR GB IT LI NL SE

(71) Applicant: HOECHST-ROUSSEL PHARMACEUTICALS INCORPORATED
Route 202-206 North
Somerville New Jersey 08876(US)

(72) Inventor: Tegeler, John J.
40 Highland Avenue
Bridgewater, N.J. 08807(US)
Inventor: Diamond, Craig J.
Eagle Rock Village Bldg. 9, Apt. 2B
Budd Lake, N.J. 07828(US)

(74) Representative: Becker, Heinrich Karl Engelbert, Dr. et al
HOECHST AKTIENGESELLSCHAFT Central Patent Department P.O. Box 80 03 20
D-6230 Frankfurt am Main 80(DE)

## (54) (Isoxazol-3-yl)arylmethanones, a process for their preparation and their use as medicaments.

(57) There are disclosed novel compounds having the

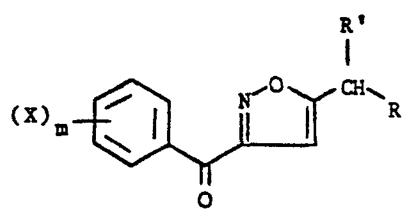

formula where each X is independently hydrogen, halogen - (F, Cl, Br or I), loweralkyl or loweralkoxy; m is 1 or 2; R' is hydrogen or loweralkyl; and R is

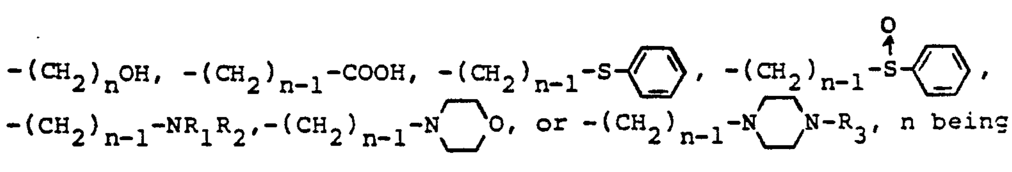

1, 2 or 3, each of $R_1$ and $R_2$ being independently hydrogen or loweralkyl and $R_3$ being

EP 0 189 771 A2

$-(CH_2)_2OH$, 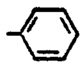, 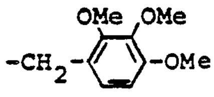, 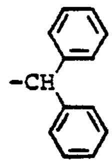 or 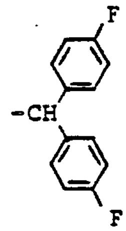

an optical antipode thereof or a pharmaceutically acceptable acid addition salt thereof, which are useful as antihypertensive, analgesic and antiinflammatory agents, methods for synthesizing them, and pharmaceutical compositions comprising an effective amount of such a compound.

# (ISOXAZOL-3-YL)ARYLMETHANONES, A PROCESS FOR THEIR PREPARATION AND THEIR USE AS MEDICAMENTS

This invention relates to novel compounds of the formula

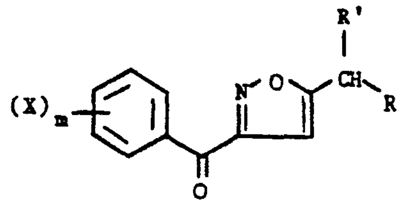

(I)

where each X is independently hydrogen, halogen (F, Cl, Br or I), loweralkyl or loweralkoxy; m is 1 or 2; R' is hydrogen or loweralkyl; and R is

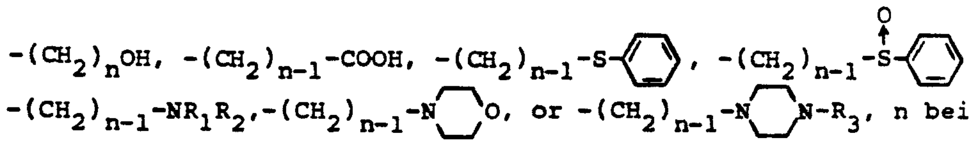

n being 1, 2 or 3, each of $R_1$ and $R_2$ being independently hydrogen or loweralkyl and $R_3$ being

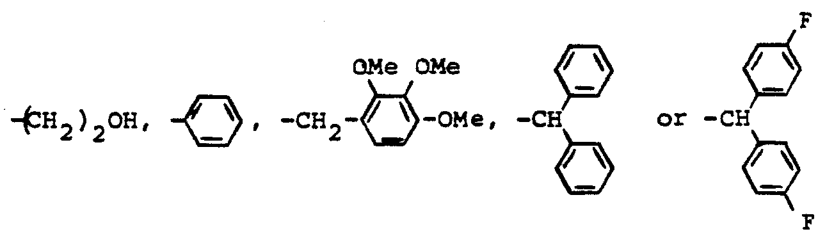

an optical antipode thereof or a pharmaceutically acceptable acid addition salt thereof, which are useful as antihypertensive, analgesic and antiinflammatory agents, methods for synthesizing them, and pharmaceutical compositions comprising an effective amount of such a compound.

Unless otherwise stated or indicated, the term loweralkyl donates a straight or branched alkyl group having from 1 to 6 carbon atoms. Examples of said loweralkyl include methyl, ethyl, n-propyl, iso-propyl, n-butyl, iso-butyl, sec-butyl, t-butyl and straight- and branched-chain pentyl and hexyl.

Unless otherwise stated or indicated, the term loweralkoxy denotes a straight or branched alkoxy group having from 1 to 6 carbon atoms. Examples of said loweralkoxy include methoxy, ethoxy, n-propoxy, iso-propoxy, n-butoxy, iso-butoxy, sec-butoxy, t-butoxy and straight- and branched chain pentoxy and hexoxy.

Unless otherwise stated or indicated, the term halogen shall mean fluorine, chlorine, bromine or iodine.

The compounds of the present invention are prepared by following one or more of the steps described below. Throughout the description of the synthetic steps, the definitions of X, m, R', R, $R_1$, $R_2$, $R_3$ and n are as given above unless otherwise stated or indicated.

## STEP A

A compound of Formula IV below is prepared by a cyclo-addition reaction between a compound of Formula II and an alcohol of Formula III.

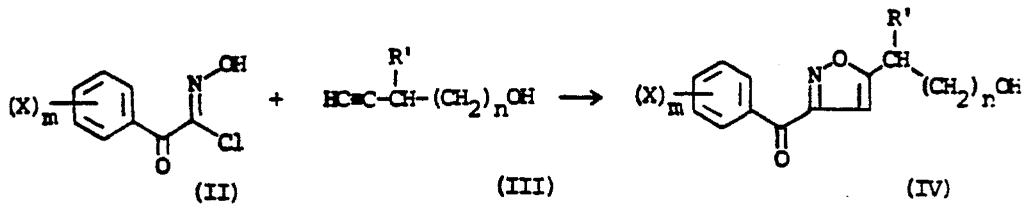

(II) (III) (IV)

Typically, said cyclo-addition reaction is conducted at a temperature of 100-150°C in a suitable medium such as an excess of said alcohol of Formula III.

STEP B

A compound of Formula V below is prepared by oxidizing compound IV with a suitable oxidizing agent including hexavalent chromium compounds such as $K_2Cr_2O_7$, $Na_2Cr_2O_7$ and $CrO_3$, and heptavalent manganese compounds such as $KMnO_4$.

IV

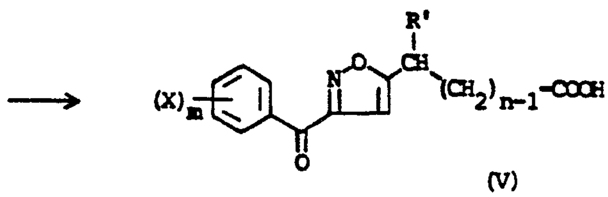

(V)

Typically, said oxidation reaction is conducted in the presence of pulverized $K_2Cr_2O_7$, concentrated sulfuric acid and a catalytic amount of $Bu_4NHSO_4$ in a suitable solvent such as dichloromethane, chloroform or the like at a temperature of about 0-50°C.

STEP C

A compound of Formula VI is prepared by the cyclo-addition reaction between compound II and a compound of Formula VII.

II +

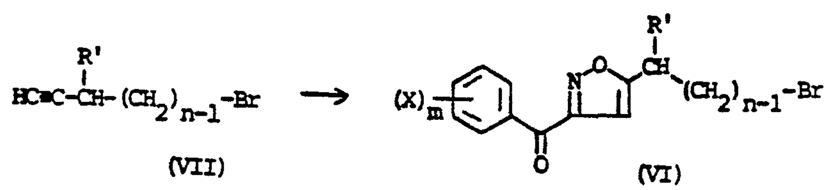

(VII) (VI)

Typically, said cyclo-addition reaction is conducted in a suitable solvent including aromatic hydrocarbons such as toluene, benzene, xylene or the like at a temperature of about 80-130°C.

STEP D

A compound of Formula VIII below is prepared by reacting compound VI with phenyl mercaptan.

VI +

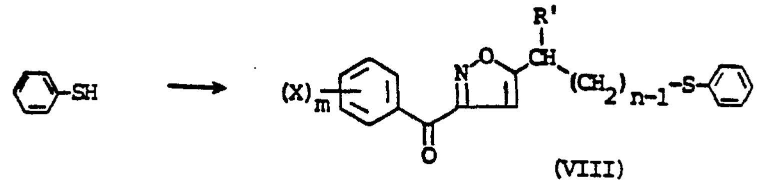

(VIII)

Said reaction is usually conducted in the presence of a base such as triethylamine, pyridine, sodium hydride or the like in a suitable solvent including acetone, ethereal compounds such as diethyl ether, tetrahydrofuran, dioxane or the like, and dimethylformamide at a temperature of about 20-60°C.

STEP E

A compound of Formula IX below is prepared by oxidizing compound VII with a suitable peroxy compound such as, for instance, m-chloroperoxybenzoic acid.

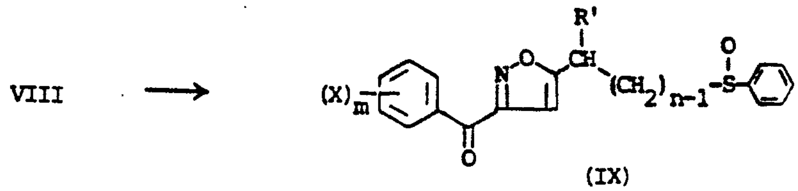

VIII (IX)

Typically, said oxidation reaction is conducted in a suitable solvent such as dichloromethane at a low temperature of from about -80°C to about -60°C.

STEP F

A compound of Formula X below is prepared by reacting compound VI with an amine of the formula $HNR_1R_2$.

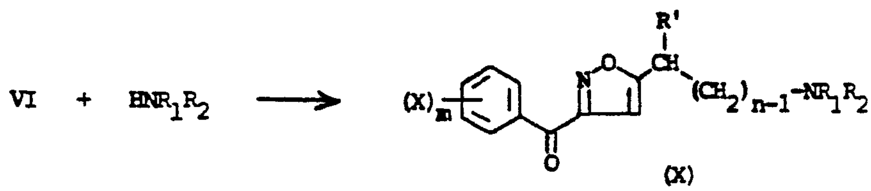

VI + $HNR_1R_2$ (X)

Typically, when said amine is a gas as in the case of dimethylamine, the amine gas is bubbled into a suitable solvent including alcohols such as methanol, ethanol and propanol, ethereal compounds such as diethyl ether, tetrahydrofuran and dioxane and mixtures thereof, in order to saturate the solvent with the amine and then the resultant saturated solution is added to a solution of compound VI in a suitable solvent such as those mentioned above. When said amine is a liquid, the reaction mixture is prepared simply by dissolving the two reactants in a suitable solvent such as those mentioned above. The reaction is usually conducted at a temperature of about 20-100°C.

STEP G

A compound of Formula XI below is prepared by reacting compound VI with morpholine.

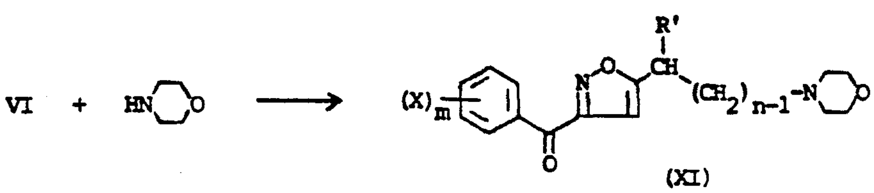

VI + (XI)

Said reaction is conducted in substantially the same manner as described in STEP F.

STEP H

A compound of Formula XII below is prepared by reacting compound VI with a compound of Formula XIII.

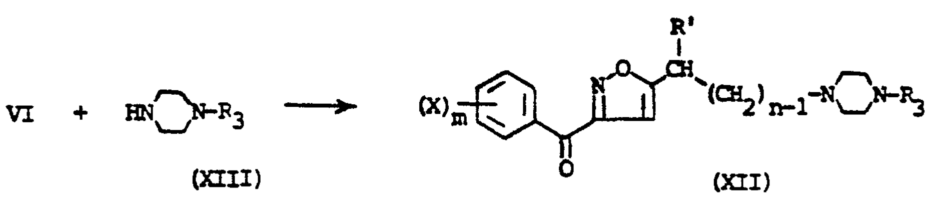

VI + (XIII) (XII)

Said reaction is conducted in substantially the same manner as described in STEP F.

In conducting the reactions described in STEPS F, G and H above, it is often convenient or advantageous to add a tertiary amine such as triethylamine and an inorganic base such as potassium carbonate or the like to the reaction medium.

The compounds of the present invention are useful as antihypertensive agents due to their ability to depress blood pressure in mammals. Antihypertensive activity is measured in the spontaneous hypertensive rat by the indirect tail cuff method described in "Methods in Pharmacology", A. Schwartz, Ed., Vol. I, Appleton-Century Crofts, New York, N.Y., 1971, p. 135. In this procedure a group of five

animals are treated orally for three days with the test compound in relation to a control group of the same number. The drop in blood pressure is measured on the third day following administration. The antihypertensive activities of some of the compounds, expressed as a decrease in mean arterial blood pressure (in mm Hg), are given in Table I along with the activity of a standard compound.

The compounds of the present invention are also useful as analgesic agents due to their ability to alleviate pain in mammals. The activity of the compounds is demonstrated in the 2-phenyl-1,4-benzoquinone-induced writhing test in mice, a standard assay for analgesia [Proc. Soc. Exptl. Biol. Med., 95, 729 (1957)]. Table I shows a result of the test of the analgesic activities of some of the compounds of this invention along with the activity of a standard compound.

The compounds of the present invention are also useful as antiinflammatory agents due to their ability to suppress inflammation in mammals. The activity of the compounds is demonstrated in the carrageenin induced rat paw edema antiinflammatory assay [Proc. Soc. Exptl. Biol. Med., III 544 (1962), J. Pharmacol. Exp., 141 (1963)]. The results of the antiinflammatory test of some of the compounds of this invention are given in Table I along with a result for a standard compound.

TABLE I

| | SHR mm dec. in BP at 50 mg/kg p.o. | CPE % dec. at 100 mg/kg p.o. | PQW % dec. |
|---|---|---|---|
| 4-Chlorophenyl-[(5-phenylsulfinyl-methyl)-isoxazol-3-yl]methanone | --- | --- | 19 at 25 mg/kg p.o. |
| 4-Chlorophenyl-[(5-phenylthio-methyl)-isoxazol-3-yl]methanone | --- | --- | 27 at 25 mg/kg p.o. |
| (5-(Morpholin-4-yl)-methylisoxazol-3-yl]-4-fluorophenylmethanone | --- | --- | $ED_{50}$ = 11.5 s.c. |
| [5-(4-Benzhydryl-piperazin-1-yl)methyl-isoxazol-3-yl]-phenyl-methanone dimaleate | 65 | --- | --- |
| [5-(4-Benzhydryl-piperazin-1-yl)methyl-3-yl]-4-chlorophenyl-methanone dimaleate | --- | --- | 90 at 20 mg/kg s.c. |
| [5-(4-Benzhydryl-piperazin-1-yl)methyl-isoxazol-3-yl]-4-fluoro-phenylmethanone dimaleate | 60 | 23 | --- |

| | | | |
|---|---|---|---|
| [5-(4-Benzhydryl-piperazin-1-yl)methyl-isoxazol-3-yl]-4-methoxyphenylmethanone dimaleate | --- | 30 | --- |
| [5-[4-(4,4'-Difluorobenzhydryl)-piperazin-1-yl]-methylisoxazol-3-yl]-4-methoxyphenyl-methanone dimaleate | 23 | --- | 48 at 20 mg/kg s.c. |
| 2-[3-(4-Toluoyl)-isoxazol-5-yl]-ethanol | --- | --- | 37 at 25 mg/kg p.o. |
| 2-(3-Benzoylisoxazol-5-yl)-ethanol | --- | --- | 54 at 25 mg/kg p.o. |
| 4-[3-(4-Chlorobenzoyl)-isoxazol-5-yl]butan-1-ol | --- | 34 | 55 at 25 mg/kg p.o. |
| 2-[3-(2,4-Dichlorobenzoyl)-isoxazol-5-yl]ethanol | --- | 29 | 59 at 25 mg/kg p.o. |
| 2-[3-(4-Chlorobenzoyl)-isoxazol-5-yl]propionic acid | --- | --- | 66 at 25 mg/kg p.o. |
| [3-(2,4-Dichloro-benzoyl)-isoxazol-5-yl]acetic acid | --- | --- | 47 at 25 mg/kg p.o. |
| [3-(4-Methoxybenzoyl)-isoxazol-5-yl]acetic acid | --- | 48 | 43 at 25 mg/kg p.o. |

| | | | |
|---|---|---|---|
| (prior art compounds) | | | |
| Methyldopa | 40 | --- | --- |
| Aspirin | | $ED_{50}$=130 mg/kg, p.o. | |
| Propoxyphene | | | $ED_{50}$ = 24.6 mg/kg, p.o. |

Effective quantities of the compounds of the invention may be administered to a patient by any of the various methods, for example, orally as in capsules or tablets, parenterally in the form of sterile solutions or suspensions, and in some cases intravenously in the form of sterile solutions. The free base final products, while effective themselves, may be formulated and administered in the form of their pharmaceutically acceptable acid addition salts for purposes of stability, convenience of crystallization, increased solubility and the like.

Acids useful for preparing the pharmaceutically acceptable acid addition salts of the invention include inorganic acids such as hydrochloric, hydrobromic, sulfuric, nitric, phosphoric and perchloric acids, as well as organic acids such as tartaric, citric, acetic, succinic, maleic, fumaric and oxalic acids.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compound, but may be varied depending upon the particular form and may conventiently be between 4% to about 70% of the weight of the unit. The amount of active compound in such compositions is such that a suitable dosage will be obtained. Preferred compositions and preparations according to the present invention are prepared so that an oral dosage unit form contains between 1.0-300 milligrams of active compound.

The tablets, pills, capsules, troches and the like may also contain the following ingredients: a binder such as micro-crystalline cellulose, gum tragacanth or gelatin; an excipient such as starch or lactose, a disintegrating agent such as alginic acid, Primogel, cornstarch and the like; a lubricant such as magnesium stearate or Sterotex; a glidant such as colloidal silicon dioxidel; and a sweetening agent such as sucrose or saccharin may be added or a flavoring agent such as pepperment, methyl salicylate, or orange flavoring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus tablets or pills may be coated with sugar, shellac, or other enteric coating agents. A syrup may contain, in addition to the active compounds, sucrose as a sweetening agent and certain preservatives, dyes, colorings and flavors. Materials used in preparing these various compositions should be pharmaceutically pure and non-toxic in the amounts used.

For the purposes of parenteral therapeutic administration, the active compounds of the invention may be incorporated into a solution or suspension. These preparations should contain at least 0.1% of active compound, but may be varied to be between 0.5 and about 30% of the weight thereof. The amount of active compound in such compositions and preparations according to the present invention are prepared so that a parenteral dosage unit contains between 0.5 to 100 milligrams of active compound.

The solutions or suspension may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in disposable syringes or multiple dose vials made of glass or plastic.

Examples of the compounds of this invention include:

2-(3-Benzoylisoxazol-5-yl)ethanol;

2-[3-(4-Fluorobenzoyl)isoxazol-5-yl]ethanol;

2-[3-(4-Chlorobenzoyl)isoxazol-5-yl]ethanol;

2-[3-(4-Toluoyl)isoxazol-5-yl]ethanol;

2-[3-(4-Methoxybenzoyl)isoxazol-5-yl]ethanol;

2-[3-(2,4-Dichlorobenzoyl)isoxazol-5-yl]ethanol;

2-[3-(4-Chlorobenzoyl)isoxazol-5-yl]propan-1-ol;

4-(3-Benzoylisoxazol-5-yl)butan-1-ol;

4-[3-(4-Chlorobenzoyl)isoxazol-5-yl]butan-1-ol;

4-[3-(4-Toluoyl)isoxazol-5-yl]butan-1-ol;

4-[3-(2,4-Dichlorobenzoyl)isoxazol-5-yl]butan-1-ol;

(3-Benzoylisoxazol-5-yl)acetic acid;

[3-(4-Fluorobenzoyl)isoxazol-5-yl]acetic acid;

[3-(4-Chlorobenzoyl)isoxazol-5-yl]acetic acid;

[3-(4-Toluoyl)isoxazol-5-yl]acetic acid;

[3-(4-Methoxybenzoyl)isoxazol-5-yl]acetic acid;

[3-(2,4-Dichlorobenzoyl)isoxazol-5-yl]acetic acid;

2-[3-(4-Chlorobenzoyl)isoxazol-5-yl]propionic acid;

4-Chlorophenyl-[(5-phenylthiomethyl)isoxazol-3-yl]-methanone;

4-Chlorophenyl-[(5-phenylsulfinylmethyl)isoxazol-3-yl]-methonone;

(5-Dimethylaminomethylisoxazol-3-yl)-4--fluorophenylmethanone;

(5-(Morpholin-4-yl)methylisoxazol-3-yl]-4--fluorophenylmethanone;

[5-(Morpholin-4-yl)methylisoxazol-3-yl]-4--chlorophenylmethanone;

[5-(4-beta-Hydroxyethylpiperazin-1-yl)methylisoxazol-3-yl]-4--fluorophenylmethanone;

[5-[(4-Phenylpiperazin-1-yl)methyl]isoxazol-3-yl]-phenylmethanone;

[5-(4-Phenylpiperazin-1-yl)methylisoxazol-3-yl]-4--fluorophenylmethanone;

[5-[4-(2,3,4-Trimethoxybenzyl)piperazin-1-yl]methylisoxazol--3-yl]-4-fluorophenylmethanone;

[5-[4-(2,3,4-Trimethoxybenzyl)piperazin-1-yl]methylisoxazol--3-yl]-4-methoxyphenylmethanone;

[5-(4-Benzhydrylpiperazin-1-yl)methylisoxazol-3-yl]-phenylmethanone;

[5-(4-Benzhydrylpiperazin-1-yl)methylisoxazol-3-yl]-4--fluorophenylmethanone;

[5-(4-Benzhydrylpiperazin-1-yl)methylisoxazol-3-yl]-4--chlorophenylmethanone;

[5-(4-Benzhydrylpiperazin-1-yl)methylisoxazol-3-yl]-4--methoxyphenylmethanone;

[5-[4-(4,4'-Difluorobenzhydryl)piperazin-1-yl]methylisoxazol--3-yl]-phenylmethanone;

[5-[4-(4,4'-Difluorobenzhydryl)piperazin-1-yl]methylisoxazol--3-yl]-4-fluorophenylmethanone;

[5-[4-(4,4'-Difluorobenzhydryl)piperazin-1-yl]methylisoxazol--3-yl]-4-chlorophenylmethanone; and

[5-[4-(4,4'-Difluorobenzhydryl)piperazin-1-yl]methylisoxazol--3-yl]-4-methoxyphenylmethanone.

The following examples are given for illustrative purposes and are not to be considered as limiting the invention disclosed herein. All temperatures are given in degrees Celcius.

EXAMPLE 1

2-(3-Benzoylisoxazol-5-yl)ethanol

A solution of 12g of phenylglyoxylohydroxamyl chloride in 30ml of 3-butyn-1-ol was refluxed under $N_2$ for 3 hours. Concentration of the product gave an oil which was purified by high pressure liquid chromatography with 5% ethyl acetate/$CH_2Cl_2$ used as an eluent to give 9.6g (67%) of an oil.

ANALYSIS:

| | | | |
|---|---|---|---|
| Calculated for $C_{12}H_{11}NO_3$: | 66.35%C | 5.11%H | 6.45%N |
| Found: | 66.49%C | 5.22%H | 6.29%N |

EXAMPLE 2

2-[3-(4-Fluorobenzoyl)isoxazol-5-yl]ethanol

A solution of 12.1g of 4--fluorophenylglyoxylohydroxamyl chloride in 30ml of 3--butyn-1-ol was refluxed under $N_2$ for 2.5 hours. Concentration of the product on a rotovapor under high-vacuum gave 17g of an oil. The residue was purified by high pressure liquid chromatography with 5% ethyl acetate/$CH_2Cl_2$ to yield 5.2g (37%) of an oil which solidified on standing, m.p. 42--45°.

ANALYSIS:

| | | | |
|---|---|---|---|
| Calculated for $C_{12}H_{10}FNO_3$: | 61.27%C | 4.29%H | 5.95%N |
| Found: | 61.30%C | 4.47%H | 5.84%N |

EXAMPLE 3

2-[3-(4-Chlorobenzoyl)isoxazol-5-yl]ethanol

A solution of 12g of 4-chlorophenylglyoxylohydroxamyl chloride in 30g of 3-butyn-1-ol was refluxed under $N_2$ for 2.5 hours. Concentration of the product on a rotovapor under high vacuum gave 17g of an oil. Purification by high pressure liquid chromatography with 5% ethyl acetate/$CH_2Cl_2$ yielded 11.8g (85%) of an oil which solidified on standing, m.p. 59-62°.

ANALYSIS:

| | | | |
|---|---|---|---|
| Calculated for $C_{12}H_{10}ClNO_3$: | 57.27%C | 4.01%H | 5.57%N |
| Found: | 57.66%C | 4.18%H | 5.39%N |

EXAMPLE 4

2-[3-(4-Toluoyl)isoxazol-5-yl]ethanol

A solution of 10g of 4-tolylglyoxylohydroxamyl chloride in 35ml of 3-butyn-1-ol was refluxed under $N_2$ for 6 hours. Concentration of the product gave an oil which was purified by high pressure liquid chromatograph with 7% ethyl acetate/$CH_2Cl_2$ used as an eluent to give 7.7g (67%) of an oil.

ANALYSIS:

| | | | |
|---|---|---|---|
| Calculated for $C_{13}N_{13}NO_3$: | 67.52%C | 5.67%H | 6.06%N |
| Found: | 67.30%C | 5.69%H | 5.69%N |

EXAMPLE 5

2-[3-(4-Methoxybenzoyl)isoxazol-5-yl]ethanol

A solution of 17.0g of 4--methoxyphenylglyoxylohydroxamyl chloride in 40ml of 3--butyn-1-ol was refluxed under $N_2$ for 4 hours. Concentration of the product on a rotovapor under a high vacuum gave 25g of an oil. The residue was purified by high pressure liquid chromatography with 15% ethyl acetate/$CH_2Cl_2$ to yield 14.1g (71%) of a oil.

ANALYSIS:

| | | | |
|---|---|---|---|
| Calculated for $C_{13}H_{13}NO_4$: | 63.14%C | 5.31%H | 5.66%N |
| Found: | 62.71%C | 5.45%H | 5.56%N |

EXAMPLE 6

2-[3-(2,4-Dichlorobenzoyl)isoxazol-5-yl]ethanol

A solution of 25.2g of 2,4--dichlorophenylglyoxylohydroxamyl chloride in 50ml of 3--butyn-1-ol was refluxed under $N_2$ for 4 hours. Concentration of the product on a rotovapor under a high vacuum gave 34g of an oil. The residue was purified by high pressure liquid chromatography with 7% ethyl acetate/$CH_2Cl_2$ to yield 19.4g of an oil which solidified on standing. 6.0g of the alcohol was again purified by high pressure liquid chromatography with 5% ethyl acetate/$CH_2Cl_2$ to yield 5.1g (58%) of a solid, m.p. 78-80°.

ANALYSIS:

| | | | |
|---|---|---|---|
| Calculated for $C_{12}H_9Cl_2NO_3$: | 50.37%C | 3.18%H | 4.89%N |
| Found: | 50.52%C | 3.20%H | 4.71%N |

EXAMPLE 7

2-[3-(4-Chlorobenzoyl)isoxazol-5-yl]propan-1-ol

A solution of 8.7g of 4-chlorophenylglyoxylohydroxamyl chloride in 16.7 g of 2-methyl-3-butyn-1-ol was refluxed under $N_2$ for 3 hours. The resulting mixture was concentrated under reduced pressure to give 13.5g of an oil. This material was purified by high pressure liquid chromatography with 5% ethyl acetate/$CH_2Cl_2$ used as an eluent to give 6.3g (59%) of an oil which solidified on standing, m.p. 49-51°.

ANALYSIS:

| | | | |
|---|---|---|---|
| Calculated for $C_{13}H_{12}ClNO_3$: | 58.76%C | 4.55%H | 5.27%N |
| Found: | 59.13%C | 4.73%H | 5.15%N |

EXAMPLE 8

4-(3-Benzoylisoxazol-5-yl)butan-1-ol

A solution of 12g of phenylglyoxylohydroxamyl chloride in 30g of 5-hexyn-1-ol was refluxed under $N_2$ for 4 hours. Concentration of the product on a rotovapor under high vacuum gave 16g of an oil. Purification by high pressure liquid chromatography with 5% ethyl acetate/$CH_2Cl_2$ used an an eluent yielded 10.0g (62%) of an oil.

ANALYSIS:

| | | | |
|---|---|---|---|
| Calculated for $C_{14}H_{15}NO_3$: | 68.55%C | 6.16%H | 5.71%N |
| Found: | 68.63%C | 6.19%H | 5.52%N |

EXAMPLE 9

4-[3-(4-Chlorobenzoyl)isoxazol-5-yl]butan-1-ol

A solution of 12g of 4-chlorophenylglyoxylohydroxamyl chloride in 30g of 5-hexyn-1-ol was refluxed under $N_2$ for 2.5 hours. Concentration of the product on a rotovapor under high vacuum gave 18g of an oil. Purification by high pressure liquid chromatography with 5% ethyl acetate/$CH_2Cl_2$ used as a eluent yielded 12.0g (78%) of an oil which solidified on standing,m.p. 48-51°.

ANALYSIS:

| | | | |
|---|---|---|---|
| Calculated for $C_{14}H_{14}ClNO_3$: | 60.11%C | 5.04%H | 5.01%N |
| Found: | 60.14%C | 5.12%H | 4.84%N |

EXAMPLE 10

4-[3-(4-Toluoyl)isoxazol-5-yl]butan-1-ol

A solution of 12g of 4-tolylglyoxylohydroxamyl chloride in 30g of 5-hexyn-1-ol was refluxed under $N_2$ for 3 hours. Concentration of the product on a rotovapor under high vacuum gave 20g of an oil. Purification by high pressure

liquid chromatography with 5% ethyl acetate/$CH_2Cl_2$ used as an eluent yielded 10.8g (68%) of an oil.

ANALYSIS:

| | | | |
|---|---|---|---|
| Calculated for $C_{15}H_{17}NO_3$: | 69.48%C | 6.61%H | 5.40%N |
| Found: | 69.38%C | 6.61%H | 5.31%N |

EXAMPLE 11

4-[3-(2,4-Dichlorobenzoyl)isoxazol-5-yl]butan-1-ol

A solution of 5.0g of 2,4-dichlorophenylglyoxylohydroxamyl chloride in 20ml of 5-hexyn-1-ol was refluxed for 2.5 hours. The solvent was evaporated in vacuo to give 7.7g of an oil. The residue was flash chromatographed twice with 0-4% ethyl acetate/$CH_2Cl_2$ to yield 4.9g (78%) of an oil.

ANALYSIS:

| | | | |
|---|---|---|---|
| Calculated for $C_{14}H_{13}Cl_2NO_3$: | 53.52%C | 4.18%H | 4.46%N |
| Found: | 53.63%C | 4.38%H | 4.20%N |

EXAMPLE 12

(3-Benzoylisoxazol-5-yl)acetic acid

To a mixture of 8.8g of 2-(3-benzoylisoxazol-5-yl)-ethanol, in 1 liter of $CH_2Cl_2$, a few crystals of tetra-n-butylammonium hydrogen sulfate and 150ml of 9M $H_2SO_4$ was added 11.9g (41 mmol) of pulverized $K_2Cr_2O_7$. The resulting mixture was stirred at room temperature for 1.5 hour. After allowing the mixture to settle, it was decanted from the unreacted dichromate. The aqueous phase was extracted with $CH_2Cl_2$, and the organics were washed with water and brine, and dried over $MgSO_4$. Concentration gave a wet solid which was triturated with cyclohexane and collected to give 7.1g of a solid. Recrystallization from toluene gave 4.0g (43%) of a solid, m.p. 108-110°.

ANALYSIS:

| | | | |
|---|---|---|---|
| Calculated for $C_{12}H_9NO_4$: | 62.34%C | 3.92%H | 6.06%N |
| Found: | 62.55%C | 3.95%H | 6.20%N |

EXAMPLE 13

[3-(4-Fluorobenzoyl)isoxazol-5-yl]acetic acid

To a mixture of 13.5g of 2-[3-(4-fluorobenzoyl)isoxazol-5-yl]ethanol and a few crystals of tetrabutylammonium hydrogen sulfate in 1 liter of $CH_2Cl_2$ was added 150ml of 9M $H_2SO_4$. To this was added 16.9g of pulverized $K_2Cr_2O_7$ and the mixture was stirred at room temperature for 1 hour. The organic phase was separated from the aqueous phase and washed with water. The acid product was extracted from the organic phase using 100ml of 5% $NaHCO_3$. The alkaline solution was washed with $CH_2Cl_2$ several times, until all of the neutral impurities were removed, and then acidified slowly with 5% HCl. The precipitated solid was collected, washed with water and dried to yield 6.7g of a powder. Recrystallization from 180ml of 50% toluene/hexane gave 5.9g (41%) of crystals, m.p. 113-115°.

ANALYSIS:

| | | | |
|---|---|---|---|
| Calculated for $C_{12}H_8FNO_4$: | 57.83%C | 3.24%H | 5.62%N |
| Found: | 57.60%C | 3.34%H | 5.57%N |

EXAMPLE 14

[3-(4-Chlorobenzoyl)isoxazol-5-yl]acetic acid

To a mixture of 8.8g of 2-[3-(4-chlorobenzoyl)isoxazol--5-yl]ethanol, 850ml of $CH_2Cl_2$, a few crystals of tetra-n--butylammonium hydrogen sulfate and 130ml of 9M $H_2SO_4$ was added 10.3g of pulverized $K_2Cr_2O_7$. The resulting mixture was stirred at room temperature for 2 hours. After allowing the mixture to settle, it was decanted from the unreacted dichromate. The aqueous phase was extracted with $CH_2Cl_2$, and the organics were washed with water and brine, and dried over $MgSO_4$. Concentration gave 5.1g of a solid. Recrystallization from toluene gave 3.0g (32%) of needles, m.p. 126-128°.

ANALYSIS:

| | | | |
|---|---|---|---|
| Calculated for $C_{12}H_8ClNO_4$: | 54.25%C | 3.04%H | 5.27%N |
| Found: | 54.08%C | 3.05%H | 5.23%N |

EXAMPLE 15

[3-(4-Toluoyl)isoxazol-5-yl]acetic acid

To a mixture of 10.7g of 2-[3-(4-toluoyl)isoxazol-5-yl]-ethanol, 1000ml of $CH_2Cl_2$, a few crystals of tetra-n--butylammonium hydrogen sulfate and 150ml of 9M $H_2SO_4$ was added 13.6g of pulverized $K_2Cr_2O_7$. The resulting mixture was stirred at room temperature for 1.5 hour. After allowing the mixture to settle, it was decanted from the unreacted dichromate. The aqueous phase was extracted with $CH_2Cl_2$, and the organics were washed with water and brine, and dried over $MgSO_4$. Concentration gave 11.0g of an oil which solidified on standing. Recrystallization from toluene gave 4.8g (43%) of a solid, m.p.105-107°.

ANALYSIS:

| | | | |
|---|---|---|---|
| Calculated for $C_{13}H_{11}NO_4$: | 63.67%C | 4.52%H | 5.71%N |
| Found: | 63.57%C | 4.49%H | 5.69%N |

EXAMPLE 16

[3-(4-Methoxybenzoyl)isoxazol-5-yl]acetic acid

To a mixture of 24.7g of 2-[3-(4-methoxybenzoyl)-isoxazol-5-yl]ethanol and a few crystals of tetrabutylammonium hydrogen sulfate in 2 liter of $CH_2Cl_2$ was added 300ml of 9M $H_2SO_4$. To this was added 29.4g of pulverized $K_2Cr_2O_7$ and the mixture was stirred at room temperature for 15 minutes. The organic phase was separated from the aqueous phase and washed with water. The acid product was extracted from the organic phase using 300ml of 3% $NaHCO_3$. The alkaline solution was washed with $CH_2Cl_2$ several times, and the solution was then acidified slowly with 5% HCl until all the acid precipitated. The solid was filtered, washed with water, and dried to give 2.6g of a powder. Recrystallization from 50ml of toluene yielded 2.3g (9%) of needles, m.p.119-121°.

ANALYSIS:

| | | | |
|---|---|---|---|
| Calculated for $C_{13}H_{11}NO_5$: | 59.76%C | 4.25%H | 5.36%N |
| Found: | 59.49%C | 4.21%H | 5.32%N |

EXAMPLE 17

[3-(2,4-Dichlorobenzoyl)isoxazol-5-yl]acetic acid

To a mixture of 12.8g of 2-[3-(2,4-dichlorobenzoyl)-isoxazol-5-yl]ethanol and a few crystals of tetra-n-butylammonium hydrogen sulfate in 1000ml of $CH_2Cl_2$ was added 150ml of 9M $H_2SO_4$. To this was added 13.2g of pulverized $K_2Cr_2O_7$ and the mixture was stirred at room temperature for 1 hour. The organic phase was separated from the aqueous phase and washed with water. The acid product was extracted from the organic phase using 400ml of 4% $NaHCO_3$. The alkaline solution was washed with $CH_2Cl_2$ several times, until all the neutral impurities were removed, and the solution was then acidified slowly with 5% HCl until all the acid precipitated. The solid was filtered, washed with water, and dried to yield 3.9g of a powder. Recrystallization from 100ml of 50% toluene/hexane gave 3.5g (26%) of crystals, m.p. 108-109°.

ANALYSIS:

| | | | |
|---|---|---|---|
| Calculated for $C_{12}H_7Cl_2NO_4$: | 48.03%C | 2.36%H | 4.67%N |
| Found: | 47.83%C | 2.44%H | 4.59%N |

EXAMPLE 18

2-[3-(4-Chlorobenzoyl)isoxazol-5-yl]propionic acid

To a mixture of 11.0g of 2-[3-(4-chlorobenzoyl)-isoxazol-5-yl]propan-1-ol in 1000ml of $CH_2Cl_2$, a few crystals of tetra-n-butylammonium hydrogen sulfate and 150ml of 9M $H_2SO_4$ was added 12.3g of pulverized $K_2Cr_2O_7$. The resulting mixture was stirred at room temperature for 1.5 hour. After allowing the mixture to settle, it was decanted from the unreacted dichromate. The aqueous phase was separated and the organics were washed with water and brine, and dried over $MgSO_4$. Concentration of the product gave 8.0g of a solid. Recrystallization from toluene with norite for decolorizing gave 2.0g of a solid, m.p. 119-123°. A second recrystallization from toluene gave 1.6g (13%) of a solid, m.p. 122.5-124.5°.

ANALYSIS:

| | | | |
|---|---|---|---|
| Calculated for $C_{13}H_{10}ClNO_4$: | 55.83%C | 3.60%H | 5.01%N |
| Found: | 55.46%C | 3.51%H | 4.95%N |

EXAMPLE 19

4-Chlorophenyl-[(5-phenylthiomethyl)isoxazol-3-yl]-methanone

A mixture of 32.7g of 4-chlorophenylglyoxylohydroxamyl chloride and 89.3g of 80% propargyl bromide in toluene was refluxed under $N_2$ overnight. The volatiles were removed by short-path distillation and the residue was recrystallized from EtOH to give 28g of a crystalline solid, m.p. 85-87.5°. This bromomethyl compound was used as such in the next step.

A solution of 12ml of $Et_3N$ in 35ml of $Et_2O$ was added dropwise to a solution consisting of 13g of the bromo compound above, 4.4ml of thiophenol and 120ml of 20% acetone/$Et_2O$. The reaction mixture was stirred at room temperature for 1 hour and allowed to stand at room temperature overnight. After dilution with $Et_2O$, the organics were washed with water, 5% aqueous HCl, saturated $NaHCO_3$ and saturated NaCl, and dried over $MgSO_4$. The solution was concentrated to give an oil. Purification by high pressure liquid chromatography using 50% $CH_2Cl_2$/hexane yielded 10.9g (48%) of an oil which solidified on standing, m.p. 54-56°.

ANALYSIS:

| | | | |
|---|---|---|---|
| Calculated for $C_{17}H_{12}ClNO_2S$: | 61.91%C | 3.67%H | 4.25%N |
| Found: | 61.52%C | 3.55%H | 4.20%N |

EXAMPLE 20

4-Chlorophenyl-[(5-phenylsulfinylmethyl)isoxazol-3-yl]-methanone

A solution of 4.45g of m-chloroperoxybenzoic acid, -(85% purity) in 100ml of $CH_2Cl_2$ was added dropwise under $N_2$ to a solution of 6.0g of 4-chlorophenyl-[(5--phenylthiomethyl)isoxazol-3-yl]-methanone in 150ml of $CH_2Cl_2$ at -70°. After 30 minutes at -70°, the reaction mixture was poured directly into a mixture of 200 ml of 10% aqueous $Na_2S_2O_3$ and 400ml of $Et_2O$. The organics were washed with saturated $NaHCO_3$ and dried over $MgSO_4$. Concentration of the product gave 5.8g of a solid which was recrystallized from acetone/hexane to yield 3.4g (55%) of a solid, m.p. 118-120°.

ANALYSIS:

| | | | |
|---|---|---|---|
| Calculated for $C_{17}H_{12}ClNO_3S$: | 59.04%C | 3.50%H | 4.05%N |
| Found: | 59.39%C | 3.54%H | 4.26%N |

EXAMPLE 21

(5-Dimethylaminomethylisoxazol-3-yl)-4--fluorophenylmethanone maleate

A 50% $Et_2O$/MeOH mixture (250ml) was saturated with dimethylamine gas and cooled in an ice-bath. To this was added a solution of 4.5g of (5-bromomethylisoxazol-3-yl)-4--fluorophenylmethanone in 25ml of $Et_2O$. The resulting solution was allowed to warm to room temperature and stirred for two days. The methanol was evaporated in vacuoto give an residue, which was dissovled in 500ml of EtOAc. The solution was washed with water until neutral and with saturated NaCl, dried over $Na_2SO_4$, and evaporated to give 3.5g of an oil which solidified on standing.

A solution of 2.8g of the amine in 30ml of $CH_2Cl_2$ was filtered and added dropwise to a solution of 1.74g maleic acid in 400ml of $Et_2O$. The precipitated salt was filtered and recrystallized from 60ml of 2-propanol to yield 3.45g (74%) of crystals, m.p. 134-135°.

ANALYSIS:

| | | | |
|---|---|---|---|
| Calculated for $C_{13}H_{13}FN_2O_2!C_4H_4O_4$: | 56.04%C | 4.71%H | 7.69%N |
| Found: | 56.18%C | 4.76%H | 7.68%N |

EXAMPLE 22

(5-(Morpholin-4-yl)methylisoxazol-3-yl]-4--fluorophenylmethanone

A solution of 4.5g of (5-bromomethylisoxazol-3-yl-4--fluorophenylmethanone in 100ml of $Et_2O$ was added dropwise to a solution of 7ml of morpholine in 300ml of 50% acetone/$Et_2O$. The resulting mixture was stirred at room temperature for 20 hours. This mixture was filtered and the volatiles evaporated to give a residue which was dissolved in 600ml of $Et_2O$. The organic solution was washed with water until it became neutral and then with saturated NaCl, and dried over $Na_2SO_4$ and concentrated in vacuo to give 4.3g of a crystalline solid. A 3.6g sample of the amine was recrystallized from 65ml of 8% toluene/hexane to yield 2.4g (64%) of crystals, m.p. 72-74°.

ANALYSIS:

| | | | |
|---|---|---|---|
| Calculated for $C_{15}H_{15}FN_2O_3$: | 62.06%C | 5.22%H | 9.65%N |
| Found: | 61.79%C | 5.15%H | 9.64%N |

EXAMPLE 23

[5-(Morpholin-4-yl)methylisoxazol-3-yl]-4--chlorophenylmethanone maleate

7.0ml of morpholine was added to a solution of 6.0g of (5-bromomethylisoxazol-3-yl)-4-chlorophenylmethanone in 100ml of 50% acetone/$Et_2O$. The resulting solution was stirred at room temperature for 2 days. This mixture was filtered and the volatiles evaporated to give a residue which was dissolved in 400ml of $Et_2O$. The organic solution was

washed with water until neutral and with saturated NaCl, dried over $Na_2SO_4$ and concentrated in vacuo to give an oil. The residue was flash chromatographed with 40% hexane/ethyl acetate to give 6.1g of an oil which solidified.

A solution of the amine in 50ml of $Et_2O$ was added dropwise to a solution of 2.6g of maleic acid in 500ml of $Et_2O$. The precipitated salt was filtered and recrystallized from 150ml of EtOH to yield 5.8g (69%) of crystals, m.p. 148-149°.

ANALYSIS:

| | | | |
|---|---|---|---|
| Calculated for $C_{15}H_{15}ClN_2O_3$!$C_4H_4O_4$: | 53.97%C | 4.54%H | 6.62%N |
| Found: | 53.89%C | 4.59%H | 6.69%N |

EXAMPLE 24

[5-(4-beta-Hydroxyethylpiperazin-1-yl)methylisoxazol-3-yl]-4--fluorophenylmethanone dimaleate

A solution of 5.7g of (5-bromomethylisoxazol-3-yl)-4--fluorophenylmethanone in 100ml of $Et_2O$ was added to a solution of 6.5g of N-beta-hydroxyethylpiperazine in 400ml of 25% acetone/$Et_2O$. The resulting mixture was stirred at room temperature for 24 hours. This mixture was filtered and the solution was concentrated in vacuoto give an oil. The residue was dissolved in 300ml of $H_2O$ and shortly thereafter a precipitate formed. The solid was filtered and dried to give 5.2g of crude solid. A solution of 4.3g of the amine in 50ml of 50% ethyl acetate/$CH_2Cl_2$ was added dropwise to a solution of excess maleic acid in 600ml of $Et_2O$. The precipitated salt was filtered and recrystallized from 160ml of isopropanol to yield 5.2g (55%) of crystals, m.p.139-140°.

ANALYSIS:

| | | | |
|---|---|---|---|
| Calculated for $C_{17}H_{20}FN_3O_3$!$2(C_4H_4O_4)$: | 53.09%C | 5.00%H | 7.43%N |
| Found: | 53.29%C | 5.01%H | 7.56%N |

EXAMPLE 25

[5-[(4-Phenylpiperazin-1-yl)methyl]isoxazol-3-yl]-phenylmethanone

A solution of 3.5g of (5-bromomethylisoxazol-3-yl)--phenylmethanone in 100ml of $Et_2O$ was added dropwise to a solution of 8.6g of phenylpiperazine in 375ml of 20% acetone/$Et_2O$. The resulting mixture was stirred at room temperature for 72 hours. This mixture was washed with water and brine, and dried over $MgSO_4$. Concentration of the product gave an oil, which was taken up in 500ml of $CH_2Cl_2$ and stirred with 20g of silica gel overnight. The silica was removed by filtration and washed with 500ml of $CH_2Cl_2$. The combined filtrates were concentrated to give 4.7g of an oil which solidified on standing. Recrystallization from ethanol yielded 3.1g (69%) of platelets, m.p. 100--102°C.

ANALYSIS:

| | | | |
|---|---|---|---|
| Calculated for $C_{21}H_{21}N_3O_2$: | 72.60%C | 6.09%H | 12.10%N |
| Found: | 72.90%C | 6.18%H | 11.95%N |

EXAMPLE 26

[5-(4-Phenylpiperazin-1-yl)methylisoxazol-3-yl]-4--fluorophenylmethanone

A solution of 3.7g of (5-bromomethylisoxazol-3-yl)-4--fluorophenylmethanone in 100ml of $Et_2O$ was added dropwise to a solution of 5.2g of N-phenylpiperazine in 400ml of 20% acetone/$Et_2O$. The resulting mixture was stirred at room temperature for 24 hours. This mixture was filtered and the solution was washed with water (2 x 200ml) and saturated NaCl, and dried over $MgSO_4$. Concentration of the product gave 5.5g of a solid which was dissolved in 100ml of $CH_2Cl_2$ and stirred with 5g of $SiO_2$. This mixture was filtered, the silica washed with $CH_2Cl_2$, and the organics were evaporated to give 3.7g of a solid. The amine was recrystallized from 135ml of 8% toluene/hexane to yield 2.5g (53%) of needles, m.p. 94-95°.

ANALYSIS:

| | | | |
|---|---|---|---|
| Calculated for $C_{21}H_{20}FN_3O_2$: | 69.02%C | 5.53%H | 11.49%N |
| Found: | 69.16%C | 5.66%H | 11.50%N |

EXAMPLE 27

[5-[4-(2,3,4-Trimethoxybenzyl)piperazin-1-yl]methylisoxazol--3-yl]-4-fluorophenylmethanone dimaleate

A solution of 3.1g of (5-bromomethylisoxazol-3-yl)-4--fluorophenylmethanone in 50ml of tetrahydrofuran was added to a solution consisting of 3.5g of N-2,3,4--trimethoxybenzylpiperazine, 1.8ml of triethylamine and 50ml of $CH_3OH$. After stirring at room temperature overnight, the mixture was concentrated to a residue. This material was flash chromatographed, using ethyl acetate as an eluent, to give 3.8g of a solid. A solution of this compound in 30ml of $CH_2Cl_2$ was added to a solution of 2.6g of maleic acid in 500ml of $Et_2O$. The collected solid was recrystallized from acetonitrile to give 4.4g (57%) of a solid, m.p. 154-155°.

ANALYSIS:

| | | | |
|---|---|---|---|
| Calculated for $C_{25}H_{28}FN_3O_5$!$2C_4H_4O_4$: | 56.49%C | 5.17%H | 5.99%N |
| Found: | 56.40%C | 5.18%H | 5.92%N |

EXAMPLE 28

[5-[4-(2,3,4-Trimethoxybenzyl)piperazin-1-yl]methylisoxazol--3-yl]-4-methoxyphenylmethanone dimaleate

A solution of 3.3g of (5-bromomethylisoxazol-3-yl)-4--methoxyphenylmethanone in 50ml of tetrahydrofuran was added to a solution consisting of 3.5g of N-2,3,4--trimethoxybenzylpiperazine, 1.8ml of triethylamine and 50ml of $CH_3OH$. After stirring at room temperature overnight, the mixture was concentrated to a residue. This material was flash chromatographed, using ethyl acetate as an eluent, to give 4.0g of an oil. A solution of this compound in 30ml of $CH_2Cl_2$ was added to a solution of 2.6g of maleic acid in 500ml of $Et_2O$. The collected solid was recrystallized from acetonitrile to give 3.6g (46%) of a solid, m.p. 147-149°.

ANALYSIS:

| | | | |
|---|---|---|---|
| Calculated for $C_{26}H_{31}N_3O_6$!$2C_4H_4O_4$: | 57.22%C | 5.51%H | 5.89%N |
| Found: | 57.21%C | 5.51%H | 5.89%N |

EXAMPLE 29

[5-(4-Benzhydrylpiperazin-1-yl)methylisoxazol-3-yl]--phenylmethanone dimaleate

A solution of 5.0g of N-benzhydrylpiperazine in 100ml of MeOH was added to a solution of 4.0g of (5--bromomethylisoxazol-3-yl)-phenylmethanone in 40ml of $Et_2O$. To this was added 2.8ml of triethylamine and the resultant solution stirred for 20 hours. The volatiles were evaporated to give a solid residue which was dissolved in 100ml of $CH_2Cl_2$. The organic solution was washed with 5% $Na_2CO_3$, water and saturated NaCl, dried over $Na_2SO_4$ and concentrated in vacuo to give 8.4g of an oil. The residue was flash chromatographed with 0.4% ethyl acetate/$CH_2Cl_2$ to yield 6.2g of amine.

A solution of the amine in 25ml of $CH_2Cl_2$ was added to a solution of 3.6g of maleic acid in 700ml of $Et_2O$. The precipitated salt was filtered and recrystallized from 600ml of isopropanol to yield 7.1g (71%) of crystals, m.p. 174--176°.

ANALYSIS:

| | | | |
|---|---|---|---|
| Calculated for $C_{28}H_{27}N_3O_2$!$2(C_4H_4O_4)$: | 64.56%C | 5.28%H | 6.27%N |
| Found: | 64.45%C | 5.28%H | 6.10%N |

EXAMPLE 30

[5-(4-Benzhydrylpiperazin-1-yl)methylisoxazol-3-yl]-4--fluorophenylmethanone dimaleate

A solution of 2.8g of (5-bromomethylisoxazol-3-yl)-4--fluorophenylmethanone in 100ml of tetrahydrofuran was added to a solution consisting of 3.0g of N--benzhydrylpiperazine, 1.7ml of triethylamine and 50ml of MeOH. The resultant solution was stirred at room temperature for 3 days. The volatiles were evaporated to give a residue which was flash chromatographed with 25% ethyl acetate/hexane to give 3.4g of an oil which solidified.

A solution of the amine in 40ml of $CH_2Cl_2$ was added to a solution of 2.0g of maleic acid in 70ml of $Et_2O$. The precipitated salt was filtered and recrystallized from 300ml of acetonitrile to yield 4.8g (70%) of crystals, m.p. 180--181°.

ANALYSIS:

| | | | |
|---|---|---|---|
| Calculated for $C_{28}H_{26}FN_3O_2 \cdot 2(C_4H_4O_4)$: | 62.87%C | 4.99%H | 6.11%N |
| Found: | 62.76%C | 4.89%H | 6.08%N |

EXAMPLE 31

[5-(4-Benzhydrylpiperazin-1-yl)methylisoxazol-3-yl]-4--chlorophenylmethanone dimaleate

A solution of 4.0g of N-benzhydrylpiperazine in 100ml of MeOH was added to a solution of 3.9g of (5--bromomethylisoxazol-3-yl)-4-chlorophenylmethanone in 30ml of tetrahydrofuran. To this was added 2.2ml of triethylamine and the solution stirred for 20 hours. The volatiles were evaporated to give a residue which was dissolved in 350ml of ethyl acetate. The solution was washed with 5% $Na_2CO_3$, water and saturated NaCl, dried over $Na_2SO_4$ and concentrated in vacuo to give a solid. The residue was flash chromatographed with 25% ethyl acetate/hexane to yield 5.8g of the amine, m.p. 137-139°.

A solution of the amine in 40ml of $CH_2Cl_2$ was added to a solution of 3.1g of maleic acid in 600ml of $Et_2O$. The precipitated salt was filtered and recrystallized from 700ml of acetonitrile to yield 7.4g (81%) of crystals, m.p. 185--186°.

ANALYSIS:

| | | | |
|---|---|---|---|
| Calculated for $C_{28}H_{26}ClN_3O_2 \cdot 2(C_4H_4O_4)$: | 61.40%C | 4.88%H | 5.96%N |
| Found: | 61.45%C | 4.96%H | 6.03%N |

EXAMPLE 32

[5-(4-Benzhydrylpiperazin-1-yl)methylisoxazol-3-yl]-4--methoxyphenylmethanone dimaleate

A solution of 2.95g of (5-bromomethylisoxazol-3-yl)-4--methoxyphenylmethanone in 30ml of tetrahydrofuran was added to a solution consisting of 3.0g of N--benzhydrylpiperazine, 1.7ml of triethylamine and 70ml of MeOH. The resultant solution was stirred at room temperature for 3 days. The volatiles were evaporated to give a residue which was flash chromatographed with 25% ethyl acetate/hexane to give 4.1g of an oil.

A solution of the amine in 30ml of $CH_2Cl_2$ was added to a solution of 2.3g of maleic acid in 500ml of $Et_2O$. The precipitated salt was filtered and recrystallized from 400ml of acetonitrile to yield 5.2g (74%) of crystals, m.p. 174--176°.

ANALYSIS:

| | | | |
|---|---|---|---|
| Calculated for $C_{29}H_{29}N_3O_3 \cdot 2(C_4H_4O_4)$: | 63.51%C | 5.34%H | 6.00%N |
| Found: | 63.48%C | 5.33%H | 6.17%N |

EXAMPLE 33

[5-[4-(4,4'-Difluorobenzhydryl)piperazin-1-yl]methylisoxazol--3-yl]-phenylmethanone dimaleate

A solution of 4.25g of (5-bromomethylisoxazol-3-yl)--phenylmethanone in 40ml of $Et_2O$ was added to a solution consisting of 5.8g of N-4,4'-difluorobenzhydrylpiperazine, 2.8ml of triethylamine and 100ml of MeOH. The resultant solution was stirred at room temperature for 16 hours. The volatiles were evaporated to give a residue which was dissolved in 150ml of ethyl acetate. The solution was washed with diluted $Na_2CO_3$, water and saturated NaCl, dried over $Na_2SO_4$ and concentrated in vacuo to give an oil. The residue was flash chromatographed with 25% ethyl acetate/hexane to give 7.6g of an oil. A solution of the amine in 40ml of $Et_2O$ was added to a solution of 4.1g of maleic acid in 700ml of $Et_2O$. The precipitated salt was filtered and recrystallized from 300ml of acetonitrile to yield 6.5g (58%) of crystals, m.p. 175-177°.

ANALYSIS:

| | | | |
|---|---|---|---|
| Calculated for $C_{28}H_{25}F_2N_3O_2!2(C_4H_4O_4)$: | 61.27%C | 4.72%H | 5.95%N |
| Found: | 61.17%C | 4.63%H | 6.01%N |

EXAMPLE 34

[5-[4-(4,4'-Difluorobenzhydryl)piperazin-1-yl]methylisoxazol--3-yl]-4-fluorophenylmethanone dimaleate

A solution of 2.8g of (5-bromomethylisoxazol-3-yl)-4--fluorophenylmethanone in 65ml of tetrahydrofuran was added to a solution consisting of 3.5g of N-4,4'--difluorobenzhydrylpiperazine, 1.7ml of triethylamine and 50ml of MeOH. The resultant solution was stirred at room temperature for 16 hours. The volatiles were evaporated to give a residue which as flash chromatographed with 25% ethyl acetate/hexane to give 4.6g of a resin.

A solution of the amine in 40ml of $CH_2Cl_2$ was added to a solution of 2.6g of maleic acid in 800ml of $Et_2O$. The precipitated salt was filtered and recrystallized from 400ml of acetonitrile to yield 4.5g (62%) of crystals, m.p. 184--186°.

ANALYSIS:

| | | | |
|---|---|---|---|
| Calculated for $C_{28}H_{24}F_3N_3O_2!2(C_4H_4O_4)$: | 59.74%C | 4.47%H | 5.80%N |
| Found: | 59.82%C | 4.51%H | 5.95%N |

EXAMPLE 35

[5-[4-(4,4'-Difluorobenzhydryl)piperazin-1-yl]methylisoxazol--3-yl]-4-chlorophenylmethanone dimaleate

A solution of 3.9ml of (5-bromomethylisoxazol-3-yl)-4--chlorophenylmethanone in 30ml of tetrahydrofuran was added to a solution consisting of 4.6g of N-4,4'--difluorobenzhydrylpiperazine, 2.2ml of triethylamine and 100ml of MeOH. The resultant solution was stirred at room temperature for 16 hours. The volatiles were evaporated to give a residue which was dissolved in 150ml of ethyl acetate. The solution was washed with diluted $Na_2CO_3$, water and saturated NaCl, dried over $Na_2SO_4$, and concentrated in vacuo to give 9.4g of an oil. The residue was flash chromatographed with 25% ethyl acetate/hexane to give 7.1g of an oil.

A solution of the amine in 40ml of $CH_2Cl_2$ was added to a solution of 3.2g of maleic acid in 900ml of $Et_2O$. The flocculent salt was filtered and recrystallized from 600ml of acetonitrile to yield 7.1g (74%) of crystals, m.p. 183-184°.

ANALYSIS:

| | | | |
|---|---|---|---|
| Calculated for $C_{28}H_{24}ClF_2N_3O_2!2(C_4H_4O_4)$: | 58.42%C | 4.37%H | 5.67%N |
| Found: | 58.48%C | 4.44%H | 5.70%N |

EXAMPLE 36

[5-[4-(4,4'-Difluorobenzhydryl)piperazin-1-yl]methylisoxazol-3-yl]-4-methoxyphenlymethanone dimaleate

A solution of 2.95g of (5-bromomethylisoxazol-3-yl)-4-methoxyphenylmethanone in 20ml of tetrahydrofuran was added to a solution consisting of 3.5g of N-4,4'difluorobenzhydrylpiperazine, 1.7ml of triethylamine and 50ml of MeOH. The resultant solution was stirred at room temperature for 3 days. The volatiles were evaporated to give a residue which was flash chromatographed with 25% ethyl acetate/hexane to give 3.7g of an oil.

A solution of the amine in 20ml of $CH_2Cl_2$ was added to a solution of 2.3g of maleic acid in 600ml of $Et_2O$. The precipitated salt was filtered and recrystallized from 220ml of acetonitrile to yield 4.5g (61%) of crystals, m.p. 175-176°.

ANALYSIS:

| | | | |
|---|---|---|---|
| Calculated for $C_{29}H_{27}F_2N_3O_3 \cdot 2(C_4H_4O_4)$: | 60.40%C | 4.80%H | 5.71%N |
| Found: | 60.70%C | 4.77%H | 5.87%N |

**Claims**

1. A compound having the formula

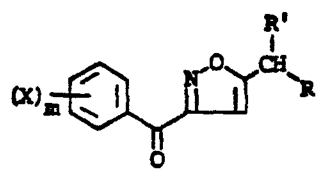

where each X is independently hydrogen, halogen, loweralkyl or loweralkoxy; m is 1 or 2; R' is hydrogen or loweralkyl; and R is $-(CH_2)_nOH$, $-(CH_2)_{n-1}-COOH$,

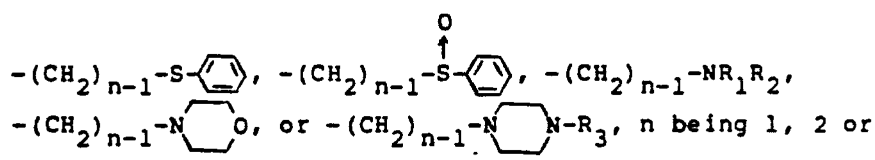

n being 1, 2 or 3, each of $R_1$ and $R_2$ being independently hydrogen or loweralkyl and $R_3$ being $-(CH_2)_2OH$,

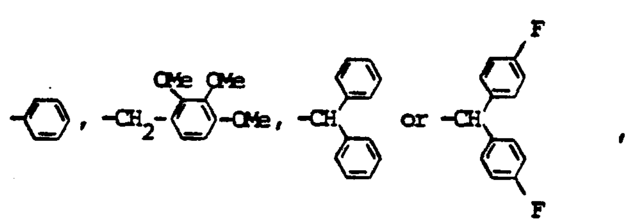

an optical antipode thereof, or a pharmaceutically acceptable acid addition salt thereof.

2. A compound as defined in claim 1, where R is $-(CH_2)_n OH$,

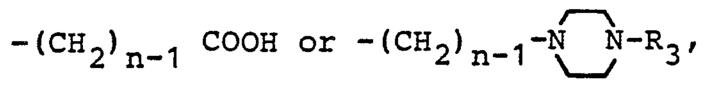

wherein $R_3$ is the group

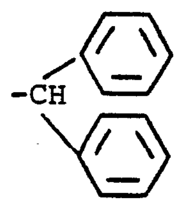

or

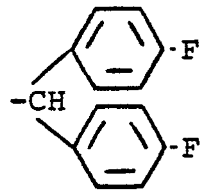

3. A compound as defined in claim 2 where R' is hydrogen and X is hydrogen, chlorine, fluorine, methyl or methoxy.

4. The compound as defined in claim 3 which is [5-(4--Benzhydrylpiperazin-1-yl)methyl-3-yl]-4--chlorophenylmethanone or a pharmaceutically acceptable acid addition salt thereof.

5. The compound as defined in claim 3 which is [5-(4--Benzhydrylpiperazin-1-yl)methylisoxazol-3-yl]--phenylmethanone or a pharmaceutically acceptable acid addition salt thereof.

6. The compound as defined in claim 3 which is [5-(4--Benzhydrylpiperazin-1-yl)methylisoxazol-3-yl]-4--fluorophenylmethanone or a pharmaceutically acceptable acid addition salt thereof.

7. The compound as defined in claim 3 which is 2-(3--Benzoylisoxazol-5-yl)-ethanol or a pharmaceutically acceptable acid addition salt thereof.

8. The compound as defined in claim 3 which is 4-[3-(4--chlorobenzoyl)-isoxazol-5-yl]butan-1-ol or a pharmaceutically acceptable acid addition salt thereof.

9. The compound as defined in claim 3 which is 2-[3-(2,4--Dichlorobenzoyl)-isoxazol-5-yl]ethanol or a pharmaceutically acceptable acid addition salt thereof.

10. The compound as defined in claim 3 which is 2-[3-(4--Chlorobenzoyl)-isoxazol-5-yl]propionic acid or a pharmaceutically acceptable acid addition salt thereof.

11. A pharmaceutical composition which comprises as active ingredient a compound as defined in claim 1 in association with a pharmaceutically acceptable carrier.

12. Use of a compound as defined in claim 1 for the preparation of a medicament having antihypertensive, analgesic and antiinflammatory activity.

13. A process for the preparation of a compound of the formula I

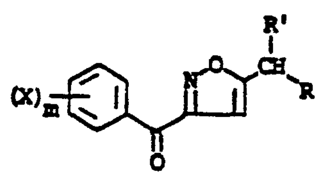

where each X is independently hydrogen, halogen, loweralkyl or loweralkoxy; m is 1 or 2; R' is hydrogen or loweralkyl; and R is $-(CH_2)_nOH$, $-(CH_2)_{n-1}-COOH$,

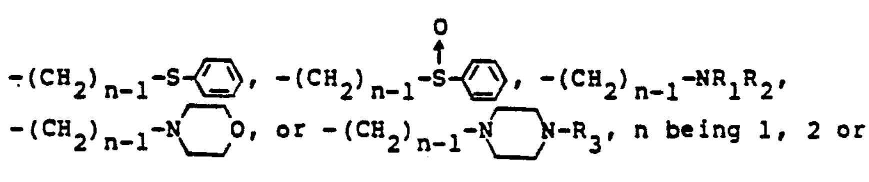

3, each of $R_1$ and $R_2$ being independently hydrogen or loweralkyl and $R_3$ being $-(CH_2)_2OH$,

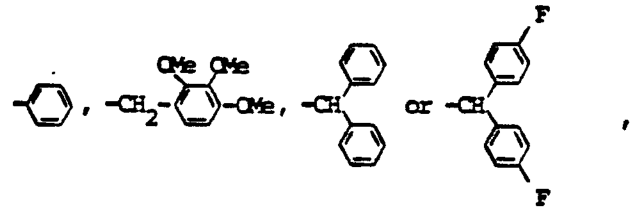

an optical antipode thereof, or a pharmaceutically acceptable acid addition salt thereof, which comprises

a) reacting a compound of the formula II

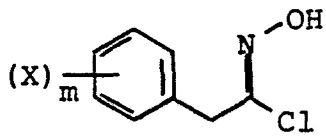

(II)

wherein X and m are as defined above, with a compound of the formula III

$$HC \equiv C - \overset{R'}{\overset{|}{CH}} - (CH_2)_nOH$$

wherein R' and n are as defined above, to form a compound of the formula I wherein R is $-(CH_2)_n-OH$, and

b) optionally oxidizing a compound of the formula I wherein R is $-(CH_2)_n-OH$ to form a compound of the formula I wherein R is $-(CH_2)_{n-1}-COOH$, or

c) reacting a compound of the formula VI

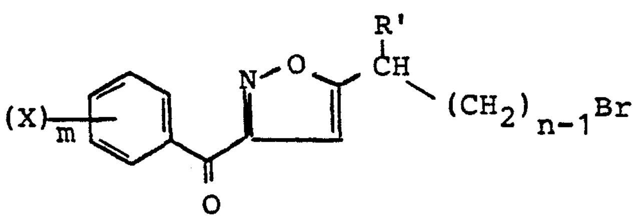

VI

wherein X, R', m and n are as defined above, with phenylmercaptan to form a compound of the formula I

wherein R is $-(CH_2)_{n-1}-S-$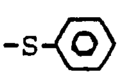 , and

d) optionally oxidizing a compound of the formula I,

wherein R is $-(CH_2)_{n-1}-S-$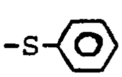 to form a compound

of the formula I wherein R is

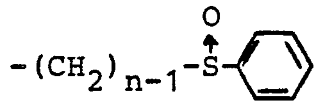, or

e) reacting a compound of the formula VI with an amine of the formula $HNR_1R_2$, where $R_1$ and $R_2$ are as defined above, to form a compound of the formula I, wherein R is --$(CH_2)_{n-1}$-$NR_1R_2$, or

f) reacting a compound of the formula VI with morpholine to form a compound of the formula I wherein R is

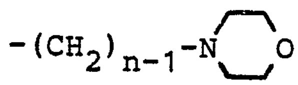, or

g) reacting a compound of the formula VI with a compound of the formula XIII

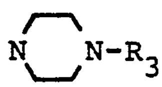 XIII

wherein $R_3$ is as defined above, to form a compound of the formula I wherein R is

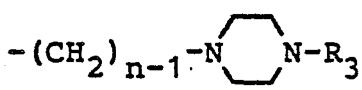

h) and, optionally prepare pharmaceutically acceptable acid addition salts thereof.

14. A process as defined in claim 13 which comprises steps a), b) or g), to form compounds of the formula I wherein R is one of the groups $-(CH_2)_nOH$, $-(CH_2)_{n-1}COOH$

or 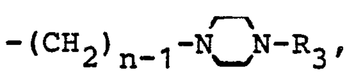, where n is as defined above and

$R_3$ is the group

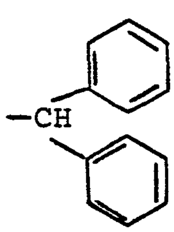 or 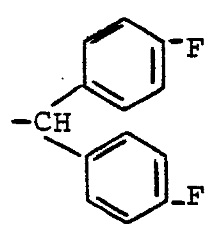

15. A process as defined in claim 14 where R' is hydrogen and X is hydrogen, chlorine, fluorine, methyl or methoxy.

Claims for contracting state : AT

1. A process for the preparation of a compound of the formula I

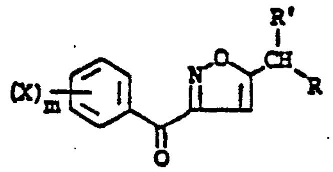

where each X is independently hydrogen, halogen, loweralkyl or loweralkoxy; m is 1 or 2; R' is hydrogen or loweralkyl; and R is $-(CH_2)_nOH$, $-(CH_2)_{n-1}-COOH$,

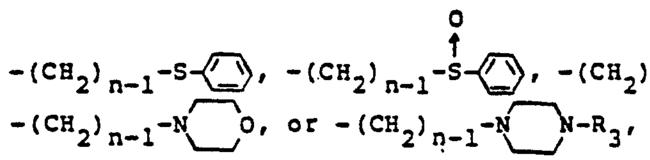 n being 1, 2 or

3, each of $R_1$ and $R_2$ being independently hydrogen or loweralkyl and $R_3$ being $-(CH_2)_2OH$,

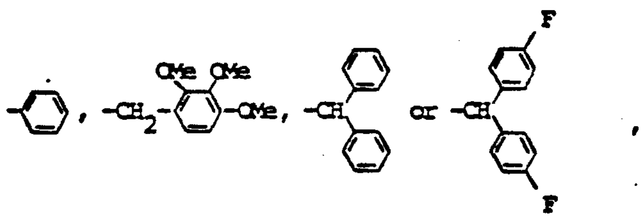

an optical antipode thereof, or a pharmaceutically acceptable acid addition salt thereof, which comprises

a) reacting a compound of the formula II

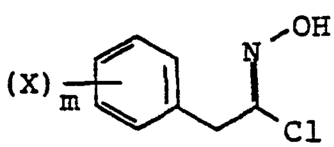

(II)

wherein X and m are as defined above, with a compound of the formula III

$$HC \equiv C - \overset{\overset{\textstyle R'}{|}}{CH} - (CH_2)_nOH$$

wherein R' and n are as defined above, to form a compound of the formula I wherein R is $-(CH_2)_n-OH$, and

b) optionally oxidizing a compound of the formula I wherein R is $-(CH_2)_n-OH$ to form a compound of the formula I wherein R is $-(CH_2)_{n-1}-COOH$, or

c) reacting a compound of the formula VI

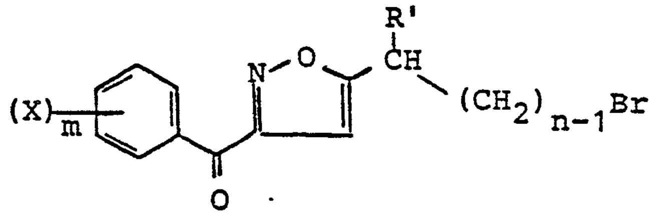

VI

wherein X, R', m and n are as defined above, with phenylmercaptan to form a compound of the formula I

wherein R is $-(CH_2)_{n-1}-S-$ 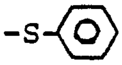 , and

d) optionally oxiding a compound of the formula I,

wherein R is $-(CH_2)_{n-1}-S-$ 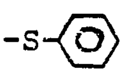 to form a compound

of the formula I wherein R is

$-(CH_2)_{n-1}-$ 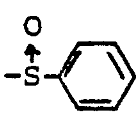 , or

e) reacting a compound of the formula VI with an amine of the formula $HNR_1R_2$, where $R_1$ and $R_2$ are as defined above, to form a compound of the formula I, wherein R is $-(CH_2)_{n-1}-NR_1R_2$, or

f) reacting a compound of the formula VI with morpholine to form a compound of the formula I wherein R is

$-(CH_2)_{n-1}-$ 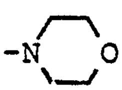 , or

g) reacting a compound of the formula VI with a compound of the formula XIII

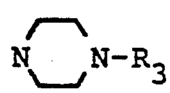

XIII

wherein $R_3$ is as defined above, to form a compound of the formula I wherein R is

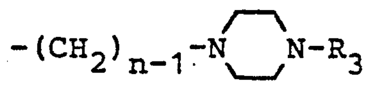

h) and, optionally prepare pharmaceutically acceptable acid addition salts thereof.

2. A process as defined in claim 13 which comprises steps a), b) or g), to form compounds of the formula I wherein R is one of the groups $-(CH_2)_n OH$, $-(CH_2)_{n-1}COOH$

or 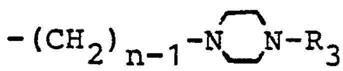, where n is as defined above and

$R_3$ is the group

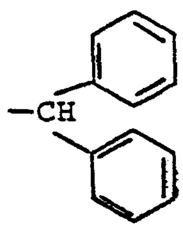

or

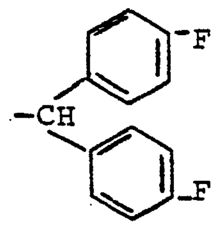

3. A process as defined in claim 14 where R' is hydrogen and X is hydrogen, chlorine, fluorine, methyl or methoxy.